Europäisches Patentamt

**European Patent Office**

Office européen des brevets

(19)

(11) **EP 1 602 912 A1**

(12) **EUROPEAN PATENT APPLICATION**
published in accordance with Art. 158(3) EPC

(43) Date of publication:
**07.12.2005 Bulletin 2005/49**

(51) Int Cl.⁷: **G01M 13/00**, G01N 15/08

(21) Application number: **04714465.4**

(22) Date of filing: **25.02.2004**

(86) International application number:
**PCT/JP2004/002223**

(87) International publication number:
**WO 2004/077006 (10.09.2004 Gazette 2004/37)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **28.02.2003 JP 2003055000**

(71) Applicants:
• **Kage, Tsuyoshi**
  **Kashiwa-shi, Chiba 2770052 (JP)**
• **Youtec Co. Ltd.**
  **Nagareyama-shi, Chiba 270-0156 (JP)**
• **Nippon API Co. Ltd.,**
  **Yokohama-shi, Kanagawa 223-0066 (JP)**

(72) Inventors:
• **KAGE, Tsuyoshi;**
  **Kashiwa-shi, Chiba 2770052 (JP)**
• **KOBAYASHI, Takumi; Sun-life-family 2a,**
  **Yamanashi 4093866 (JP)**
• **MIZOKAMI, Kazuaki; C/o Nippon Api Co., ltd.**
  **Yokohama-shi, Kanagawa 2230066 (JP)**
• **KOBAYASHI, Akiyoshi; C/o Nippon Api Co., Ltd.**
  **Yokohama-s hi, Kanagawa 2230066 (JP)**

(74) Representative: **Bunke, Holger**
  **Prinz & Partner GbR**
  **Manzingerweg 7**
  **81241 München (DE)**

(54) **METHOD OF MEASURING GAS BARRIER PROPERTY OF PLASTIC MOLDING**

(57)    Object of present invention to overcome a problem of a dispersion of an evaluation of gas barrier property depending on absorbed moisture of plastic, and carry out measurements in which a measurement value response is good and accuracy is good, wherein from the beginning of a container the gas barrier property of a plastic molded body does not depend on the amount of moisture absorbed thereof. A method of measuring the gas barrier property of the plastic molded body such as a plastic container, a plastic sheet or a plastic film or the like which uses a gas analyzer to measure the amount of permeation of a measurement object gas permeating through the plastic molded body, comprising: a heat drying process which heats and dries said plastic molded body in a temperature range which does not cause deformation or heat deterioration.

Fig.3

EP 1 602 912 A1

**Description**

**TECHNOLOGICAL FIELD**

The present invention is related to a method of measuring a gas barrier property which evaluates the gas barrier property of a plastic molded body quickly with good accuracy, and is a measuring method adapted for quality control of the gas barrier property when mass producing gas barrier property plastic containers.

**PRIOR ART TECHNOLOGY**

A gas barrier property plastic container is disclosed in Japanese Laid-Open Patent Application No. HEI 8-53116, for example. In Japanese Laid-Open Patent Application No. HEI 8-53116, with regard to the oxygen barrier property of a plastic container, the oxygen permeability was measured at 40°C using an OX-TRANTWIN manufactured by Modem Control Company. Further, with regard to the carbon dioxide gas barrier property, the carbon dioxide gas permeability was measured at 25°C using a PERMATRANC-4 Model manufactured by Modern Control Company.

The gas barrier property measurement of a plastic molded body such as a plastic container, a plastic sheet or a plastic film or the like mainly uses the method disclosed in Japanese Laid-Open Patent Application No. HEI 8-53116. In the case of a plastic container, because the thickness is thick and the shape of the container is complex, a period of about one week is required until the measurement value stabilizes. Accordingly, up to now there has not been a method which makes it possible to quickly evaluate the gas barrier property.

Further, Japanese Laid-Open Patent Application No. HEI 11-118763 discloses technology for a method of measuring hydrogen gas concentration, for example, as an invention which measures gas concentration with high accuracy by atmospheric pressure ionization mass spectrometry.

In the technology of Japanese Laid-Open Patent Application No. HEI 11-118763, instead of measuring hydrogen directly, water (water vapor) is created from hydrogen, and then by measuring this water, a hydrogen concentration conversion is obtained. As described in line 10 of the right column on page 2 of the specification of Japanese Laid-Open Patent Application No. HEI 11-118763, the reason water is created is because of the special problems that arise in mass spectrometry due to the fact that hydrogen does not ionize efficiently into a parent ion ($H_2+$, mass 2).

A test which evaluates the gas barrier property using an atmospheric pressure ionization mass spectrometer (APIMS) has not been developed yet.

**SUMMARY OF THE INVENTION**

**PROBLEMS TO BE SOLVED BY THE INVENTION**

As described in Japanese Laid-Open Patent Application No. HEI 8-53116, a gas permeability measuring device measures oxygen or carbon dioxide separately. In the experience of the present inventors, the time it takes to measure the gas permeability using the apparatus disclosed in Japanese Laid-Open Patent Application No. HEI 8-53116 requires at least one week for the measurement value to stabilize in the case where the oxygen permeability is measured, for example. Accordingly, if the accuracy of the measurement value is to be made higher, a period of time greater than one week becomes necessary in order to measure the gas barrier property of one plastic container. For the reason that a long period of time is required for measurement in this way, the present inventors assumed that in Japanese Laid-Open Patent Application No. HEI 8-53116, from the viewpoint of securing measurement accuracy, because the flow rate of the carrier gas which carries the permeated measurement object gas can not be made larger, a long period of time is required due to the fact that the circulation of gas inside the container is poor and the response of the measurement value is slow.

Further, there are plastic resins that have a large hygroscopicity depending on the type, for example, and the resin of polyethylene terephthalate (PET) containers has a large hygroscopicity, wherein 100 g of resin absorbs 0.3 - 0.5 g of water at 20°C at a relative humidity of 60 - 80%. The present inventors discovered that there are effects on the detector when water molecules absorbed inside the resin are present, and the measurement value of the oxygen permeability had large variations depending on the water content of the resin.

After plastic containers are formed, in the case where the containers are stored in a warehouse, the containers will absorb moisture. In the case where quality confirmation of the gas barrier property is carried out in a plastic container forming plant after a period of time has passed since the containers were formed, and in the case where the gas barrier property of the plastic containers forms one quality confirmation subject when a beverage bottling plant receives the plastic containers, the detector receives effects depending on the difference of the amount of absorbed moisture, whereby the evaluation of the gas barrier property becomes dispersed.

It is an object of the present invention to overcome the problem of the dispersion of the evaluation of the gas barrier property depending on the absorbed moisture of plastic described above, and carry out measurements in which the measurement value response is good and accuracy is good, wherein from the beginning of the container the gas barrier property of the plastic molded body does not depend on the amount of moisture absorbed thereof.

Namely, it is an object of the present invention to provide a method of measuring a gas barrier property which improves the accuracy of gas barrier property measurements by heating a plastic molded body in a temperature range which does not cause deformation or heat deterioration to dry the plastic molded body before measuring a measurement object gas permeated through the plastic molded body, wherein preferably the absorbed water is removed by heat drying, and the accuracy of gas barrier property measurements is improved by quickly adjusting the plastic molded body for which the effects of water have been eliminated. Further, this provides a method of measuring a gas barrier property which makes the detection of the amount of permeation of the measurement object gas respond with good response and high accuracy and drastically shortens the measurement time by using a mass spectrometer, in particular an atmospheric pressure ionization mass spectrometer, and flowing a relatively large amount of carrier gas. At this time, it is an object to measure not just one measurement object gas, but a plurality of measurement object gases simultaneously with good accuracy.

Further, it is an object of the present invention to provide a method of measuring a gas barrier property of a plastic molded body, in particular a plastic container or a gas barrier property plastic container wherein one of the inner surface or the outer surface or both surfaces thereof are coated with a gas barrier property thin film.

It is an object of the present invention to provide a method of measuring the oxygen gas barrier property of a plastic molded body, and in particular a method of measuring the oxygen gas barrier property depending on permeation of oxygen from an air atmosphere.

It is an object of the present invention to provide a method which can measure the gas barrier property of one of oxygen gas, carbon dioxide gas or hydrogen gas or a combination thereof with good accuracy while eliminating the effects of impurity gases by creating an atmosphere containing a measurement object gas from a reference gas of measurement object gas - argon or a reference gas of measurement object gas - nitrogen gas which is adjusted so that the measurement object gas forms a prescribed pressure, wherein the measurement object gas is one of oxygen gas, carbon dioxide gas or hydrogen gas or a combination thereof.

Further, it is an object of the present invention to provide a method of measuring gas barrier property which completely eliminates the effects of oxygen gas present inside the plastic resin before measurement by using oxygen gas of mass number 18 as the measurement object gas, and separately measuring the oxygen gas (oxygen gas of mass number 16 is 99.762%, oxygen gas of mass number 17 is 0.038%, and oxygen gas of mass number 18 is 0.200%, wherein oxygen gas of mass number 16 forms the major portion) present inside the plastic resin before measurement and the oxygen gas permeating through the plastic resin at the time of measurement. Inside the plastic resin, permeation occurs due to diffuse permeation depending on the concentration gradient of the gas, but the initial data of a normal measuring method detects as a measurement value both the oxygen gas present inside the plastic resin before measurement and the oxygen gas permeating by diffuse permeation. In the present invention, it can be expected that the true measurement value of the gas barrier property is quickly detected by detecting only the oxygen gas permeating by diffuse permeation from the beginning of measurement.

Further, it is an object of the present invention to provide a measuring method which combines at least one gas from oxygen gas of mass number 16, carbon dioxide gas or hydrogen gas with oxygen gas of mass number 18 to form measurement object gases, and can carry out simultaneous measurements of the gas barrier property of a plurality of gas types.

In the case where a gas barrier property plastic container is mass produced, instead of corresponding to only one coating chamber for coating the container with a gas barrier property thin film, the reality is that a plurality of chambers are operated simultaneously or are operated alternately in sequence. In this regard, it is another object of the present invention to provide a method of measuring a gas barrier property which evaluates the gas barrier property of each container when plastic containers are coated with gas barrier property thin films in a mass production state using a plurality of coating chambers, and understands the operating state of the coating chambers by comparing the gas barrier properties of the containers which underwent film formation in each coating chamber. This is because there are many cases where the quality of the gas barrier thin film created by film formation depends on whether or not the coating chamber is operating normally. In particular, because the present invention can quickly measure the gas barrier property, it is expected that it is possible to judge the quality evaluation of the gas barrier properties of containers on a production line by the result of the present measuring method. The prior art measuring method which requires an evaluation time of one week or more takes too much time as an evaluating method in a production line.

It is an object of the present invention to quickly carry out gas substitution to quickly reach a steady state which makes it possible to begin measurements by setting the flow rate of argon gas or nitrogen gas supplied to an argon gas atmosphere side or a nitrogen gas atmosphere side at or above a prescribed rate in an atmosphere adjusting process and a measurement object gas measuring process.

## MEANS FOR SOLVING THE PROBLEMS

In view of the problems described above, through diligent development to design higher measurement accuracy by controlling the dispersion of measurement data due to water molecules and design high speed measurements by efficiently carrying out gas substitution required for measurements for a plastic molded body, the present inventors achieved the present invention by discovering that the problems described above are solved efficiently by (1) heat drying the plastic molded body before measuring the gas permeability, and (2) preferably using a mass spectrometer, in particular an atmospheric pressure ionization mass spectrometer even though it is possible to achieve a higher accuracy and a higher speed using various gas measuring methods if heat drying of the plastic molded body is made a precondition.

Namely, the method of measuring the gas barrier property of a plastic molded body according to the present invention is a method of measuring the gas barrier property of a plastic molded body such as a plastic container, a plastic sheet or a plastic film or the like which uses a gas analyzer to measure the amount of permeation of a measurement object gas permeating through the plastic molded body, and includes a heat drying process which heats and dries said plastic molded body in a temperature range which does not cause deformation or heat deterioration. The method of measuring the gas barrier property of a plastic molded body according to the present invention includes the case where such method includes an atmosphere adjusting process which forms an atmosphere including a measurement object gas at one side of the wall surfaces of said plastic molded body, and forms a carrier gas atmosphere which carries said measurement object gas after permeation to said gas analyzer at the other side forming a front and back relationship of said wall surfaces. In this regard, the carrier gas atmosphere is preferably an argon atmosphere or a nitrogen gas atmosphere. In this regard, in order to shorten the period of time required for measurements, said heat drying process is preferably carried out at the same time in said atmosphere adjusting process. Further, the method of measuring the gas barrier property of a plastic molded body according to the present invention includes the case where such method includes a measurement object gas measuring process which measures the amount of permeation of said measurement object gas permeating through said plastic molded body from one side of said wall surfaces toward the other side with said gas analyzer at the time when said amount of permeation of said measurement object gas reaches a roughly steady state.

In the method of measuring the gas barrier property of a plastic molded body according to the present invention, said heat drying process includes a process which removes water absorbed by said plastic molded body. Further, said heat drying process includes a process which substitutes adsorption gas adsorbed on one side of the wall surfaces of said plastic molded body with gas of the atmosphere including said measurement object gas, and substitutes adsorption gas adsorbed on the other side forming a front and back relationship of the wall surfaces of said plastic molded body with gas of said carrier gas atmosphere.

Further, in the method of measuring the gas barrier property of a plastic molded body according to the present invention, the gas analyzer is preferably a mass spectrometer, and more preferably an atmospheric pressure ionization mass spectrometer.

In the method of measuring the gas barrier property of a plastic molded body according to the present invention, when said plastic molded body is a plastic container or a gas barrier property plastic container in which a gas barrier property thin film is coated on one of the inner surface or the outer surface or both surfaces thereof, preferably an atmosphere including a measurement object gas is formed outside the container and said carrier gas atmosphere is formed inside the container in said atmosphere adjusting process.

In this regard, in the method of measuring the gas barrier property of a plastic molded body according to the present invention, preferably said measurement object gas is oxygen gas, and the atmosphere including said measurement object gas is an air atmosphere.

Alternatively, preferably said measurement object gas is one of oxygen gas, carbon dioxide gas or hydrogen gas or a combination thereof, and the atmosphere including said measurement object gas is a measurement object gas - argon atmosphere or a measurement object gas - nitrogen gas atmosphere in which adjustments are carried out so that said measurement object gas forms a prescribed partial pressure.

Alternatively, preferably the measurement object gas is oxygen gas of mass number 18, and the atmosphere including said measurement object gas is an oxygen gas of mass number 18 - argon atmosphere or an oxygen gas of mass number 18 - nitrogen gas atmosphere in which adjustments are carried out so the oxygen gas of mass number 18 forms a prescribed partial pressure.

Alternatively, preferably the measurement object gas is a mixed gas of oxygen gas of mass number 18 and at least one gas from oxygen gas of mass number 16, carbon dioxide gas or hydrogen gas, and the atmosphere including said measurement object gas is a measurement object gas - argon atmosphere or a measurement object gas - nitrogen gas atmosphere in which adjustments are carried out so that said measurement object gas forms a prescribed partial pressure.

Further, in the method of measuring the gas barrier property of a plastic molded body according to the present invention, preferably said plastic container is a gas barrier property plastic container which is mass produced by arranging a plurality of coating chambers for coating the inner surface or the outer surface of the plastic container or both surfaces thereof with a gas barrier property thin film and operating said coating chambers simultaneously or sequentially, said heat drying process, said atmosphere adjusting process and said measurement object gas measuring process are carried out at a level where a prescribed number of film formations is carried out in each of said coating chambers, and then a chamber operation judgment process which judges poor operation of said coating chambers by comparing the gas barrier properties of said gas barrier property plastic containers in each of said coating chambers is carried out.

Further, in the method of measuring the gas barrier property of a plastic molded body according to the present invention, preferably the argon flow rate per 1 minute or the nitrogen flow rate per 1 minute supplied to said carrier gas atmosphere side is greater than or equal to twice the volume of said plastic container in said atmosphere adjusting process and said measurement object gas measuring process.

**EFFECT OF THE INVENTION**

The present invention makes it possible to overcome the problem of the dispersion of the evaluation of the gas barrier property depending on the absorbed moisture of plastic described above, and carry out measurements in which the measurement value response is good and accuracy is good, wherein from the beginning of the container the gas barrier property of the plastic molded body does not depend on the amount of moisture absorbed thereof. Namely, the accuracy of gas barrier property measurements can be improved by quickly adjusting the plastic molded body for which the effects of water have been eliminated. Further, it was possible to provide a method of measuring a gas barrier property which makes the detection of the amount of permeation of the measurement object gas respond with good response and high accuracy and drastically shortens the measurement time by preferably using a mass spectrometer, and more preferably an atmospheric pressure ionization mass spectrometer as the gas analyzer, and flowing a relatively large amount of carrier gas. Further, evaluation was possible in several hours even in a plastic molded body having a complex shape such as a plastic container or a gas barrier property plastic container wherein one of the inner surface or the outer surface or both surfaces thereof are coated with a gas barrier property thin film. At this time, it was possible to measure not just one measurement object gas, but a plurality of measurement object gases simultaneously with good accuracy. The measurement object gas is oxygen gas, carbon dioxide gas, hydrogen gas and a combination of these. Further, by using oxygen gas of mass number 18 as the measurement object gas, it was possible to provide a gas barrier property measuring method which completely eliminates the effects of oxygen gas present inside the plastic resin before measurements. Further, it was possible to provide a method of measuring a gas barrier property which evaluates the gas barrier property of each container when plastic containers are coated with gas barrier property thin films in a mass production state using a plurality of coating chambers, and understands the operating state of the coating chambers by comparing the gas barrier properties of the containers which underwent film formation in each coating chamber.

**BRIEF DESCRIPTION OF THE DRAWINGS**

Fig 1 is a conceptual structure drawing showing one embodiment of a coating apparatus of a mass production machine.

Fig. 2 is a conceptual drawing showing one embodiment of a coating chamber arrangement of a coating apparatus.

Fig. 3 is a schematic drawing showing one embodiment of an apparatus which measures the gas barrier property of a plastic container.

Fig. 4 is a graph which shows the changes in oxygen gas concentration and hydrogen gas concentration depending on the measurement elapsed time for Specific Examples 1 and 2.

Fig. 5 is a graph which shows the relationship between the ionic strength and the mass number at the time of the background measurement of the operating procedure (1) of the gas barrier property measuring method in Specific Examples 1 and 2.

Fig. 6 is a graph which shows the relationship between the ionic strength and the mass number at the time of the oxygen gas concentration measurement of Specific Example 1.

Fig. 7 is a graph which shows the relationship between the ionic strength and the mass number at the time of the hydrogen gas concentration measurement of Specific Example 2.

Fig. 8 is a graph which shows the changes in oxygen gas concentration and hydrogen gas concentration depending on the measurement elapsed time for Specific Examples 3 and 4.

Fig. 9 is a graph which shows the relationship between the ionic strength and the mass number at the time of the background measurement of the operating procedure (1) of the gas barrier property measuring method in Specific Examples 3 and 4.

Fig. 10 is a graph which shows the relationship between the ionic strength and the mass number at the time of the oxygen gas concentration measurement of Specific Example 3.

Fig. 11 is a graph which shows the relationship between the ionic strength and the mass number at the time of the hydrogen gas concentration measurement of Specific Example 4.

Fig. 12 is a graph which shows the relationship between the film thickness of the DLC film and the amount of oxygen gas permeation.

Fig. 13 is a graph which shows the relationship between the film thickness of the DLC film and the amount of hydrogen gas permeation.

Fig. 14 is a graph which shows the relationship between the measurement day number and the amount of oxygen gas permeation at such times for Comparative Examples 4~8.

Fig. 15 is a graph showing the change in oxygen concentration inside the carrier gas flowing through the inside of a PET container, and shows the case where there is a heat drying process and the case where there is none.

Fig. 16 is a graph showing the change in oxygen concentration inside the carrier gas flowing through the inside of a polyethylene container, and shows the case where there is a heat drying process and the case where there is none.

The meaning of the symbols is as follows. 1 is a liquid argon tank, 2 is a liquid argon vaporizer, 3, 22 are pressure reducing valves, 4 is a getter, 5 is a bypass line, 6, 9, 23 are mass flow controllers, 7, 10, 14 are purifiers, 8 is a PET bottle line, 11 is a PET bottle, 12 is valve B, 13 is valve A, 15 is an APIMS, 16 is a gas supply pipeline, 17 is a gas exhaust pipeline, 21 is a standard gas cylinder, 24 is PET bottle sealing means, and 31 is PET bottle outside atmosphere adjusting means.

**PREFERRED EMBODIMENTS OF THE INVENTION**

Detailed descriptions showing embodiments and specific examples of the present invention are given below, but it should not be interpreted that the present invention is limited to these descriptions.

The plastic molded body according to the present invention can be illustrated by a plastic container, a plastic sheet or a plastic film. In the present embodiment, a description is given for the example of a plastic container, in particular a beverage container. In the case where the gas barrier property of a film thickness of several tens of $\mu$m is measured for a plastic film, because the film thickness is thin, there are instances where the measurement is completed in a relatively short period of time. In the case where a plastic container forms the measurement object, because the resin thickness is 0.3 - 1 mm, a quick measurement is required compared with the case of a film. Further, because a beverage container has a three-dimensional shape with a complicated shape inside the container, a long period of time is required to flow the carrier gas and carry out gas substitution completely. Accordingly, it is an object for which it is difficult to evaluate the gas barrier property at high speed and high accuracy. The present invention is not influenced by the shape of the container, and the use of the container can be illustrated by carbonated beverages such as beer and the like, fruit juices, nutrient drinks, and medicines.

The resin used when forming the plastic container of the present invention can be illustrated by polyethylene terephthalate (PET) resin, polyethylene terephthalate type copolyester resin (a copolymer named PETG which uses cyclohexane dimethanol in place of ethylene glycol for the alcohol component of polyester, made by Eastman Chemical), polybutylene terephthalate resin, polyethylene naphthalate resin, polyethylene (PE) resin, polypropylene (PP) resin, cycloolefin copolymer (COC, cyclic olefin copolymer) resin, ionomer resin, poly-4-methylpentene-1 resin, polymethyl methacrylate resin, polystyrene (PS) resin, ethylene-vinyl alcohol copolymer resin, polyacrylonitrile resin, polyvinyl chloride resin, polyvinylidene chloride (PVDC) resin, polyamide resin, polyamide-imide resin, polyacetal resin, polycarbonate (PC) resin, polysulfone resin, or ethylene tetrafluoride resin, acrylonitrile-styrene resin, polyether sulfone resin, and acrylonitrile-butadiene-styrene resin. Of these, PET is particularly preferred. In the present invention, a description is given for a PET bottle as an example of a plastic container.

The percentage of water absorption of the plastic (24 hours, 23°C) is divided into three classifications.

(1) Material which is difficult to absorb moisture, PE: < 0.01%, PP: < 0.005%, PS: 0.04~0.06%, PVDC: trace.

(2) Intermediate material, PET: 0.3 - 0.5%, PC: 0.35%, polyether sulfone: 0.3 - 0.4%.

(3) Material having high moisture absorbance, polyimide: 2.9%, nylon 6: 9.5%, cellulose acetate: 5 - 9%.

The plastic molded body according to the present invention may be a gas barrier property plastic container wherein one of the inner surface or the outer surface or both surfaces thereof are coated with a gas barrier property thin film. The gas barrier property thin film which coats the wall surfaces of the plastic container can be illustrated by SiOx, DLC (diamond like carbon), DLC containing Si, polymer like carbon, aluminum oxide, polymer like silicon nitride, and acrylic acid resin coating. Of these, DLC is particularly preferred because it has a superior oxygen barrier property and water vapor barrier property, is chemically inactive, can be disposed in the same way as plastic because carbon and hydrogen form the main components, and has the ability to follow the expansion and contraction of plastic because it is flexible. In the present invention, the DLC film is a film called an i-carbon film or a hydrogenated amorphous carbon film (a - C : H), and also includes a hard carbon film. Further, a DLC film is an amorphous state carbon film, and includes SP$^3$ bonding. A hydrocarbon gas such as acetylene, for example, is used as a source gas for forming the DLC film, and a Si-containing hydrocarbon gas is used as a source gas for forming a Si-containing DLC film. By forming this kind of DLC film on one of the inner surface or the outer surface of the container or on both surfaces thereof, a container is obtained which can be used as a one-way container or a returnable container for carbonated beverages and sparkling beverages and the like.

Further, the gas barrier property thin film can be coated on the inner surface or the outer surface or both thereof, but the forming of a gas barrier property thin film on the inner surface is preferred in view of securing a gas barrier property and preventing either the contents from being absorbed into the plastic or minute quantities of components contained inside the plastic from dissolving into the contents and the fact that it is economical. Further, as for the method of forming a DLC film, there is the film forming method described in Laid-Open Patent Application No. HEI 8-53116, for example.

The method of measuring a gas barrier property of a plastic molded body according to the present invention is a method which carries out measurements of the amount of permeation of a measurement object gas permeating through the plastic molded body with a gas analyzer, which includes at least (1) a heat drying process which heats and dries the plastic molded body in a temperature range which does not cause deformation or heat deterioration. This heat drying process may include a process which removes water absorbed by the plastic molded body. Further, the heat drying process may include a process which substitutes the adsorption gas adsorbed on one side of the wall surfaces of the plastic molded body with a gas atmosphere containing a measurement object gas, and substitutes the adsorption gas adsorbed on the other side forming a front and back relationship of the wall surfaces of the plastic molded body with a gas of a carrier gas atmosphere such as argon, nitrogen gas or the like.

The method of measuring a gas barrier property of a plastic molded body according to the present invention is a method further includes (2) an atmosphere adjusting process which forms an atmosphere including a measurement object gas at one side of the wall surfaces of the plastic molded body, and forms a carrier gas atmosphere such as argon, nitrogen gas or the like on the other side forming a front and back relationship of the wall surfaces. This carrier gas is a gas which carries said measurement object gas after permeation to said gas analyzer, and may contain 0.5 - 3.0% hydrogen gas for removing oxygen as impurities. The hydrogen gas for removing oxygen as impurities reacts by catalyzing oxygen as impurities inside the carrier gas before making contact with the plastic molded body. On the other hand, because there is no reaction with oxygen after contact with the catalyst, there is no reaction with permeated oxygen.

The method of measuring a gas barrier property of a plastic molded body according to the present invention is a method further includes (3) a measurement object gas measuring process which measures the amount of permeation with various gas analyzers, preferably a mass spectrometer, and more preferably an atmospheric pressure ionization mass spectrometer when the measurement object gas permeates through the plastic molded body from one side of the wall surfaces to the other side and the amount of the permeated measurement object gas forms a roughly steady state, namely when the detector value of the concentration of the measurement object gas reaches a prescribed value.

The present inventors discovered that when water molecules due to absorbed moisture and the like forming a measurement object are present inside the molecular structure of the plastic molded body, the water molecules will have effects on the detector, and this will cause the measurement value of oxygen, carbon dioxide and the like to become unstable, but on the other hand it is possible to eliminate the adverse effects of water molecules by removing the water molecules absorbed in the plastic molded body. For example, PET resin absorbs 0.3 - 0.5 g of water per 100 g. In the case where a beverage bottling plant or the like receives plastic containers that have been coated with a gas barrier property thin film, it is normal procedure to carry out a quality inspection of whether or not the containers have prescribed gas barrier properties. In this regard, there are many cases where moisture absorption occurs in a gas barrier property thin film coated plastic container. If a measurement of the gas barrier property of this container is carried out as is, a measurement error will be included depending on the moisture absorption state of the container. In the present invention, preferably a heat drying process is provided before measuring the gas barrier property. The heat drying process is carried out for the purpose of removing water molecules absorbed in the plastic molded body, eliminating the adverse effects of water molecules on the detector, and reducing the effect of adsorption gas adsorbed on the surface of the plastic molded body. Further, as means for removing the water molecules absorbed in the plastic molded body, means which continuously flow large quantities of dry argon gas or dry nitrogen gas through the plastic molded body have been conceived. However, because the diffusion rate of water molecules depends on the temperature, it is understood that about one week is required to remove most of the water molecules from the resin. In comparison with this, the removal of water molecules can be carried out at high speed by heat drying. At this time, preferably gas substitution with dry argon or dry nitrogen gas is continuously carried out inside the plastic container at the same time heat drying is carried out. This is not for the purpose of drying the resin with dry argon or dry nitrogen gas, but is instead for the purpose of carry out exhaust with dry argon or dry nitrogen gas as a carrier gas for the water molecules that diffuse and are released from the inside of the resin to the outside of the resin, and for this reason there is no need to flow a large quantity of dry argon or dry nitrogen gas. Further, the APIMS which is the detector used in the present invention requires a heat drying process because it is easy to receive effects from water molecules.

In the heat drying process, the plastic molded body is heated in a temperature range which does not cause deformation or heat deterioration. Blowing hot gas of dry argon or dry nitrogen gas in the container is effective for carrying out quick drying, but when heating is carried out at a high temperature above the vicinity of the glass transition point, the container will shrink and create deformations. Accordingly, in the present invention, preferably drying is carried out at or below a temperature level where shape deterioration and thin film deterioration such as peeling, cracking and like of the gas barrier property thin film do not occur. For example, for a PET bottle, preferably heating is carried out at 60 - 80°C.

The atmosphere adjusting process provides a difference in concentration of measurement object gas on the front surface and the back surface near the wall surfaces in a front and back relationship of the plastic molded body, and this is provided to carry out diffusion of gas from the high concentration side to the low concentration side. In this regard, in the atmosphere adjusting process, the heat drying process may be carried out at the same time. The atmosphere adjusting process requires a little time until stabilizing, and the heat drying process requires a little time for heating and cooling, but these can be carried out at the same time, and this leads to a shortening of the time required for measurements.

When taking a plastic container as an example, preferably the outside of the plastic container forms an atmosphere containing a measurement object gas, and the inside of the container forms a carrier gas atmosphere such as an argon atmosphere or a nitrogen gas atmosphere. In this case, the measurement object gas concentration outside the container is a high concentration, the measurement object gas concentration inside the container forms a low concentration, and the measurement object gas diffuses and permeates through the inside of the resin from the outer surface of the container toward the inner surface of the container. Further, the plastic container may be a gas barrier property plastic container wherein one of the inner surface or the outer surface or both surfaces thereof are coated with a gas barrier property thin film.

In one embodiment which measures the oxygen gas barrier property, oxygen gas forms the measurement object gas, and an air atmosphere forms the atmosphere containing the measurement object gas. In order to carry the permeated oxygen gas to the detector, argon gas or nitrogen gas is flowed as a carrier gas at a flow rate of 500 - 2000 ml/min, for example. Argon gas or nitrogen gas forms a substitution gas until a steady state is formed.

In the present embodiment, because a lowering of the measurement accuracy is brought about when water molecules are present in the measurement path, preferably water molecules are removed as much as possible. Namely, preferably high purity argon or high purity nitrogen gas is used as a carrier gas, and a purifier is provided directly after the argon supply source or the nitrogen gas supply source to remove water molecules.

In an embodiment in which the oxygen gas barrier property, the carbon dioxide gas barrier property or the hydrogen gas barrier property is measured separately or these are combined to measure the gas barrier property at the same time, the measurement object gas is formed by one of oxygen gas, carbon dioxide gas, or hydrogen gas or a combination of these. Then, the atmosphere containing the measurement object gas is formed by a measurement object gas - argon atmosphere or a measurement object gas - nitrogen gas atmosphere in which the measurement object gas is adjusted to form a prescribed partial pressure. In this case, in order to create a measurement object gas - argon atmosphere, oxygen gas - argon gas, carbon dioxide gas - argon gas, hydrogen gas - argon gas, oxygen gas - carbon dioxide gas - argon gas, oxygen gas - hydrogen gas - argon gas, hydrogen gas - carbon dioxide gas - argon gas, or oxygen gas - carbon dioxide gas - hydrogen gas - argon gas is prepared as a standard gas by adjusting each gas to have a prescribe partial pressure, and then this standard gas is flowed through the gas path. Alternatively, in order to create a measurement object gas - nitrogen gas atmosphere, oxygen gas - nitrogen gas, carbon dioxide gas - nitrogen gas, hydrogen gas - nitrogen gas, oxygen gas - carbon dioxide gas - nitrogen gas, oxygen gas - hydrogen gas - nitrogen gas, hydrogen gas - carbon dioxide gas - nitrogen gas, or oxygen gas - carbon dioxide gas - hydrogen gas - nitrogen gas is prepared as a standard gas by adjusting each gas to have a prescribe partial pressure, and then this standard gas is flowed through the gas path. Compared with the case where the atmosphere containing the measurement object gas is formed by an air atmosphere at the time the gas barrier property of oxygen gas is measured, because the effects of impurity gases such as water vapor in particular are eliminated, an improvement of measurement accuracy can be expected.

In another embodiment which measures the oxygen gas barrier property, the measurement object gas is formed by oxygen gas of mass number 18, and the atmosphere containing the measurement object gas is formed by an oxygen gas of mass number 18 - argon atmosphere in which the oxygen gas of mass number 18 is adjusted to form a prescribed partial pressure or is formed by an oxygen gas of mass number 18 - nitrogen gas atmosphere in which the oxygen gas of mass number 18 is adjusted to form a prescribed partial pressure. The reason for using oxygen gas of mass number 18 is as follows. Normal oxygen gas is mass number 16, and APIMS which is the detector of atmospheric pressure ionization spectrometry can detect the difference in mass number. On the other hand, molecular properties such as gas permeation and the like are the same because they are the same element. Because the proportion of oxygen gas of mass number 18 present inside the plastic resin from the beginning is extremely small at 0.200%, it is possible to separate the detection error due to diffuse permeation of oxygen gas (mass number 16) present inside the plastic resin from the beginning by measuring oxygen gas of mass number 18 as a measurement object gas. Namely, if oxygen gas of mass number 18 is measured, the state where permeation through the plastic molded body begins is understood in real time.

In the applied embodiment of the case where oxygen gas of mass number 18 is used as a measurement object gas, a measurement object gas is formed by combining at least one gas of oxygen gas of mass number 16, carbon dioxide gas or hydrogen gas with oxygen gas of mass number 18, and as for the atmosphere containing the measurement object gas, a standard gas of a measurement object gas - argon atmosphere in which the measurement object gas is adjusted to form a prescribed partial pressure, or a standard gas of a measurement object gas - nitrogen gas atmosphere in which the measurement object gas is adjusted to form a prescribed partial pressure is prepared and such atmosphere is created. In this case, in addition to making it possible to separate the detection error due to diffuse permeation of the oxygen gas (mass number 16) present inside the plastic resin from the beginning, it becomes possible to carry out simultaneous analysis of carbon dioxide gas, oxygen gas, hydrogen gas or a combination of these gases. By using a standard gas, it is possible to eliminate the effects of impurity gases.

The composition of the standard gases of measurement object gas - argon gas and the composition of the standard gases of measurement object gas - nitrogen gas are shown in Table 1.

TABLE 1

| Standard Gas Number | Concentration of Oxygen Gas of Mass Number 16 (%) | Concentration of Oxygen Gas of Mass Number 18 (%) | Concentration of Carbon Dioxide Gas (%) | Concentration of Hydrogen Gas (%) | Concentration of Argon (%) |
|---|---|---|---|---|---|
| 1 | 21 | | | | 79 |
| 2 | | 21 | | | 79 |
| 3 | | | 4 | | 96 |
| 4 | | | | 4 | 96 |
| 5 | 21 | | 3 | 1 | 75 |
| 6 | 21 | | 3 | | 76 |
| 7 | 21 | | | 1 | 78 |
| 8 | | | 3 | 1 | 96 |

TABLE 1 (continued)

| Standard Gas Number | Concentration of Oxygen Gas of Mass Number 16 (%) | Concentration of Oxygen Gas of Mass Number 18 (%) | Concentration of Carbon Dioxide Gas (%) | Concentration of Hydrogen Gas (%) | Concentration of Argon (%) |
|---|---|---|---|---|---|
| 9 | 21 | 1 | 3 | 1 | 74 |
| 10 | | 21 | 3 | 1 | 75 |
| 11 | | 21 | | 1 | 78 |
| 12 | | 21 | 3 | | 76 |
| 13 | 21 | 1 | | | 78 |
| 14 | 21 | 1 | 3 | | 75 |
| | | | | | Concentration of Nitrogen Gas (%) |
| 15 | 21 | | | | 79 |
| 16 | | 21 | | | 79 |
| 17 | | | 4 | | 96 |
| 18 | | | | 4 | 96 |
| 19 | 21 | | 3 | 1 | 75 |
| 20 | 21 | | 3 | | 76 |
| 21 | 21 | | | 1 | 78 |
| 22 | | | 3 | 1 | 96 |
| 23 | 21 | 1 | 3 | 1 | 74 |
| 24 | | 21 | 3 | 1 | 75 |
| 2 5 | | 2 | | 1 | 78 |
| 26 | | 21 | 3 | | 76 |
| 27 | 21 | 1 | | | 78 |
| 28 | 21 | 1 | 3 | | 75 |

In the measurement object gas measuring process, the measurement object gas permeates through the plastic molded body from one side of the wall surfaces toward the other side, the permeated measurement object gas is carried to the gas detector with argon gas or nitrogen gas as a carrier gas, and the amount of permeation of the measurement object gas is determined by measuring the gas concentration of the measurement object gas at the time when the detected amount of permeation of the measurement object gas reaches a steady state. For example, in the case where a plastic container forms a measurement object, the outside of the container forms an atmosphere which includes a measurement object gas at a prescribed partial pressure. On the other hand, because the permeated measurement object gas is carried sequentially to the detector with argon gas or nitrogen gas as a carrier gas, the measurement object gas concentration (partial pressure) inside the container is close to roughly zero.

In this regard, the gas permeability coefficient P of plastic is shown by Equation 1 using the gas diffusion coefficient D inside the plastic and the gas solubility coefficient S to the plastic.

Equation 1

$$P = D \times S$$

The gas permeation amount Q of the plastic is shown by Equation 2 using the gas permeability coefficient P, the permeation area A, the permeation time t and the pressure p.

Equation 2

$$Q = P \times A \times t \times p$$

The gas permeation amount Q of the plastic can be represented by Equation 2, but in actual measurements if the concentration gradient between the measurement object gas concentration (which is a prescribed concentration) outside the plastic container and the measurement object gas concentration (the measurement object gas concentration is roughly zero as described above) inside the plastic container does not reach a steady state, measurement errors will occur. Gas molecules diffuse inside the plastic, and the time it takes for the concentration gradient of the gas molecules inside the plastic to reach a steady state is called the "lag time". As shown in Equation 3, the lag time is proportional to the square of the plastic thickness, and is inversely proportional to the diffusion coefficient.

Equation 3

$$\text{Lag Time} = (\text{Plastic Thickness})^2 / (6 \times \text{Diffusion Coefficient D})$$

In this regard, Table 2 shows the data shown in Non-Patent Document 1.

TABLE 2

| Gas | P | D | S | Ep | $E_D$ | $\sigma^2$ | $\sigma^{-2}$ |
|---|---|---|---|---|---|---|---|
| $N_2$ | 1 | 1 | 1 | 1 | 1 | 1 | 1 |
| CO | 1.2 | 1.1 | 1.1 | 1 | 1 | 0.95 | 1.05 |
| $CH_4$ | 3.4 | 0.7 | 4.9 | (1) | (1) | 0.98 | 1.02 |
| $O_2$ | 3.8 | 1.7 | 2.2 | 0.86 | 0.90 | 0.83 | 1.2 |
| He | 15 | 60 | 0.25 | 0.62 | 0.45 | 0.45 | 2.2 |
| $H_2$ | 22.5 | 30 | 0.75 | 0.70 | 0.65 | 0.55 | 1.8 |
| $CO_2$ | 24 | 1 | 24 | 0.75 | 1.03 | 1.0 | 1 |
| $H_2O$ | (550) | 5 | - | 0.75 | 0.75 | 0.94 | 1.06 |
| (Note) Other Gases are converted using 1 for Nitrogen. | | | | | | | |

(Non-Patent Document 1) D.W.Van Krevelen, Properties of Polymers, P555

As is clear from Equation 3, the diffusion coefficient D is different and the lag time is also different depending on the type of measurement object gas, but when reaching the time where each of the measurement object gases undergoing diffuse permeation inside the resin finishes permeating, the permeation of the measurement object gases can be said to have formed a steady state. However, there are also instances where the plastic molded body has a complicated shape, and because the permeation needs to reach a steady state in all parts, preferably the gas permeation is judged to have reached a steady state after a prescribed period of time has elapsed from the detection of permeation of a measurement object gas. Further, even in the state where permeation becomes steady in each part of the container, the measurement object gas remains inside the container until being carried to the detector. The gas inside the container (in this case, a carrier gas such as argon gas or nitrogen gas with the measurement object gas) reaches a fixed concentration, and after the effect of this remaining gas is eliminated, a steady state is finally reached. Accordingly, until the amount of permeation of the measurement object gas reaches a steady state, the permeation of the measurement object gas must reach a steady state and the gas concentration inside the container must become fixed.

The detection of the measurement object gas is carried out with a gas analyzer. Preferably, this is carried out by a non-deflection type mass spectrometer such as a quadrupole type mass spectrometer or a time-of-flight mass spectrometer or the like, or a deflection type mass spectrometer such as a magnetic deflection type mass spectrometer, a trochoid type mass spectrometer, an omegatron or the like. Further, preferably an atmospheric pressure ionization mass spectrometer is used. Atmospheric pressure ionization mass spectrometry (APIMS, Atmospheric Pressure Ionization Mass Spectrum) which depends on an atmospheric pressure ionization method (API, Atmospheric Pressure Ionization) is means which can analyze minute quantities of impurities, and has a special feature in the point that it can carry out measurements with high sensitivity in real time. This is due to the fact that the gas introduction portion of APIMS can be operated at atmospheric pressure. In order to plan the making of high sensitivity with a mass spectrometer, it is necessary to increase the amount of ionization (high efficiency ionization) of the detection object component, but in APIMS high efficiency ionization is achieved by two-step ionization. First, in the beginning a sample gas (main component: C, impurity: X) containing a minute quantity of impurity undergoes primary ionization by corona discharge in an ionization portion. In the primary ionization, only one portion of the sample is ionized in the same way as in many ionization methods. The composition of the created ions is roughly the same as the sample, and is formed by the main component ion $C^+$ which is the major portion and a slight amount of the impurity ion $X^+$. In an electron impact ionization method (EI) which is carried out under normal reduced pressure, because the ions created by only one-step ionization corresponding to this primary ionization are detected, the amount of the object impurity ion $X^+$ is small, and this makes it difficult to carry out high sensitivity detection. On the other hand, in the atmospheric pressure ionization method, an increase of $X^+$ is planned using the following secondary ionization. Namely, $C^+$ from the ions created by the primary ionization are useless ions for the purpose of analysis. These useless ions $C^+$ search out most of the remaining impurities X which have not been ionized yet, and this is a method of moving charges by charge-exchange reactions. These ion molecular reactions are reactions which move charges from a high ionization potential to a low ionization potential. Because the carrier gas (nitrogen, argon, hydrogen or the like) has a higher ionization potential than the impurity molecules ($H_2O$, $O_2$, organic matter and the like), it becomes possible to carry out high sensitivity analysis of impurities.

APIMS is less affected even when a large amount of carrier gas such as argon or nitrogen gas is flowed. Accordingly, gas remnants inside the plastic container are controlled by flowing a large amount of carrier gas, and it is possible to carry the permeated measurement object gas directly to the detector. In this way, the response speed of detection can be made higher, and this makes it possible to carry out detection in roughly real time. In addition, it is possible to shorten the waiting time until the gas concentration inside the container reaches a steady state. Further, because the concentration (partial pressure) of the permeated measurement object gas is inevitably lowered if there is a large amount of carrier gas, a detection method which can carry out minute quantity analysis is required. For the reason given above, there has been a demand for a method which can carry out minute quantity analysis and does not lower the detection accuracy even when a large amount of carrier gas is flowed as in APIMS.

As described above, by combining heat drying with the use of an atmospheric pressure ionization analyzer, the gas barrier property of the plastic molded body when first used as a container can be measured at high accuracy and high speed.

In the oxygen barrier property measuring apparatus represented by the OX-TRANTWIN manufactured by Modem Control Company shown in Japanese Laid-Open Patent Application No. HEI 8-53116, the measurement accuracy is lowered when a large amount of carrier gas is flowed. The carrier gas flow rate can be up to a maximum of 50 ml/min, and most measurements are at about 10 ml/min. On the other hand, in the measuring method according to the present embodiment, in the atmosphere adjusting process and the measurement object gas measuring process, the flow rate per one minute of argon gas supplied to the argon atmosphere side is preferably greater than or equal to twice the volume of the plastic container, for example, the carrier gas flow rate in a 350 ml container can be made a maximum of 2000 ml/min, and measurements are carried out at about 500 - 1000 ml/min. In this way, compared with the measuring method used in Japanese Laid-Open Patent Application No. HEI 8-53116, in the measuring method according to the present embodiment, because it is possible to flow 50 - 100 times as much carrier gas (purge gas), a steady state can be reached quickly. In particular, it is possible to control gas remnants inside the container and carry out gas substitution quickly at the time the gas barrier property of a plastic container having a complicated three-dimensional shape inside is evaluated. Even in the above-described oxygen barrier property measuring apparatus disclosed in Japanese Laid-Open Patent Application No. HEI 8-53116, it is possible to eliminate the effects of water and quickly reach a steady state by providing a heat drying process.

Further, because the gas permeability coefficient inside plastic has a temperature dependency and a humidity dependency, measurements need to be carried out at a constant temperature.

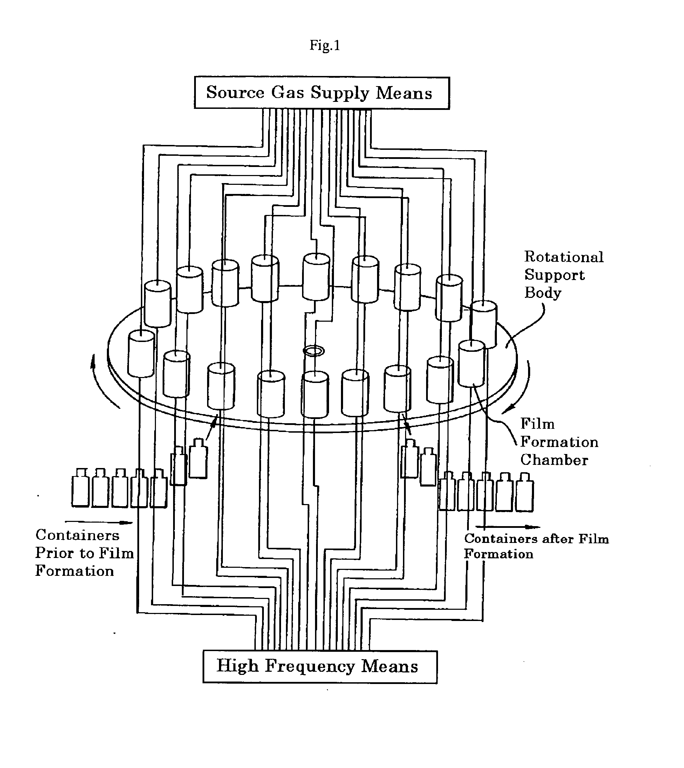

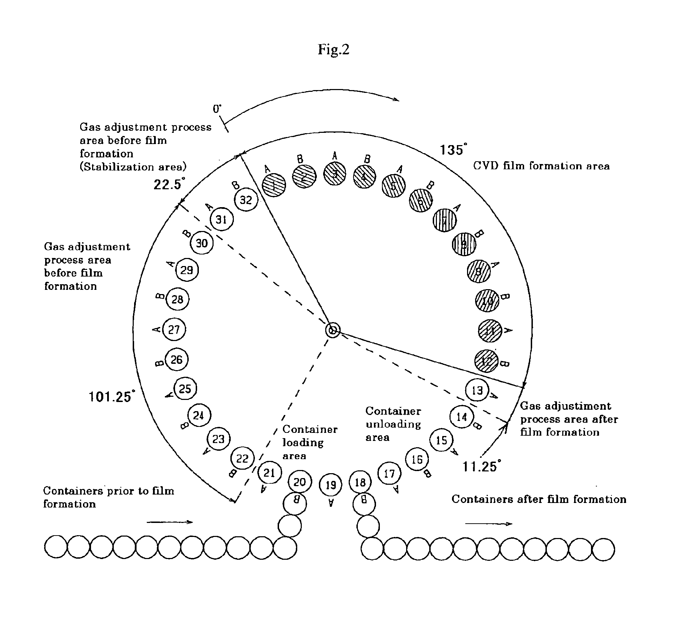

For example, as shown in Fig. 1 and Fig. 2, the method of measuring a gas barrier property of a plastic molded body according to the present invention can be applied to gas barrier property plastic containers which are manufactured by a mass production machine in which a plurality of coating chambers are arranged for coating a gas barrier property thin film on one of the inner surface or the outer surface of the plastic containers or on both thereof and coating is carried out in a mass production manner by simultaneously or sequentially operating said coating chambers. Namely, a heat drying process, an atmosphere adjusting process and a measurement object gas measuring process are carried out at a level where a prescribed number of film formations is carried out in each coating chamber, and then a chamber operation judgment process which judges poor operation of the coating chambers by comparing the gas barrier property of the gas barrier property plastic containers in each coating chamber is carried out.

Fig. 1 is a conceptual drawing showing the structure of a coating apparatus in which a plurality of coating chambers (film formation chambers) is arranged in the shape of a circle, and a gas barrier thin film is coated on one of the inner surface or the outer surface of the container or on both surfaces thereof during the period in which the circle is rotated once. Fig. 2 is a conceptual drawing showing the arrangement of coating chambers and each corresponding process in the rotating apparatus. Further, the letters A, B assigned to the coating chambers show the type of high frequency power source, wherein the high frequency power source A (not shown in the drawings) and the high frequency power source B (not shown in the drawings) supply a high frequency to the sequential coating chambers.

In this regard, a heat drying process, an atmosphere adjusting process and a measurement object gas measuring process are carried out at a level where a prescribed number of film formations is carried out in each coating chamber. Observations are carried for one specific coating chamber arranged in the circle. For example, the film formation apparatus of Fig. 1 forms a gas barrier property thin film every time the circle undergoes one rotation, and a plastic container that has undergone film formation is sampled at every 100 film formations (every time the circle undergoes 100 rotations), and the gas barrier property of said plastic container is measured by the measuring method according to the present embodiment described above. This is also carried out in the same way for the other coating chambers. The sampling proportion is appropriately determined in accordance with the manufacturing rate of the mass production machine. Next, the gas barrier properties of the gas barrier property plastic containers in each coating chamber are compared. When there is a container having a low gas barrier property compared with the average value of the gas barrier property of each container, the coating chamber corresponding to said container is assumed to have poor operation, and so the coating chamber is judged to have poor operation. In this kind of case, because there is a strong possibility that the gas barrier property thin film coated plastic container that underwent film formation in the poor operation coating chamber has a poor quality gas barrier property, quality confirmation such as the carrying out of a separate gas barrier measurement may be carried out by sampling from mass produced gas barrier property thin film coated plastic containers. Further, as long as there is a judgment of the quality of the operation of each coating chamber, measurements of the amount of gas permeation may be carried out before the amount of gas permeation completely stabilizes, and in this case, preferably the measurement conditions are standardized.

Next, before describing the specific gas barrier property measuring method of the present embodiment, a description will be given for an embodiment of a gas barrier property measuring apparatus for carrying out the present measuring method. This apparatus is shown for the purpose of describing the measuring method, and the measuring method is not limited to this apparatus structure.

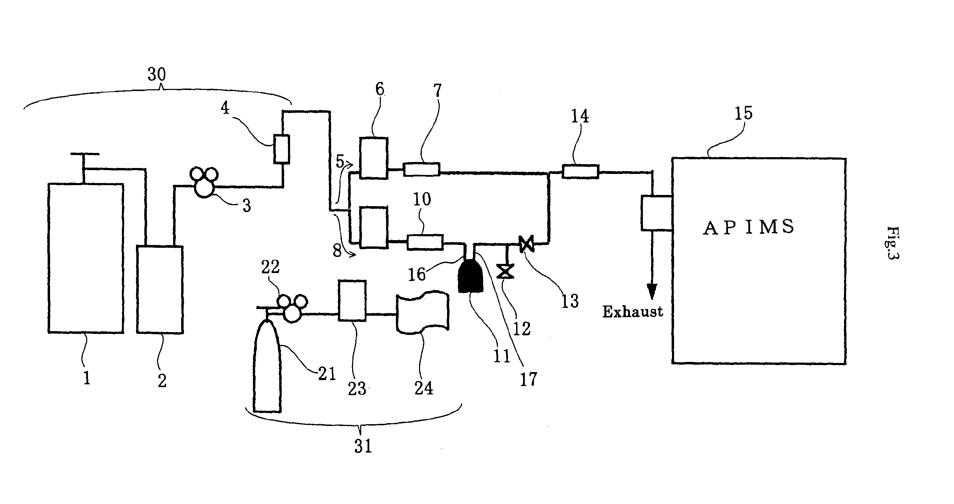

Fig. 3 shows a schematic drawing showing one embodiment of a plastic container gas barrier property measuring apparatus. The pipeline structure of the present apparatus is a measurement object gas detecting structure constructed from a liquid argon tank 1, a liquid argon vaporizer 2, a pressure reducing valve 3 and a getter 4 (GT100, manufactured by Nihon API Company) which removes impurities such as water molecules and the like connected in that order by a pipeline, and a bypass line 5 and a PET bottle (PET bottle) line 8 provided to branch in parallel downstream of the getter 4, a repurifier (MS-10J, manufactured by Nihon API Company) through which impurities pass and are removed after the bypass line 5 and the PET bottle line 8 flow together again, and an APIMS 15 (UG-240PN manufactured by Hitachi Tokyo Electronics Company).

A mass flow controller 6 and a purifier 7 are connected in that order by a pipeline to the bypass line 5, and a mass flow controller 9, a purifier 10, the inside space of a PET bottle 11, a valve B 11 and a valve A 13 are connected in that order by a pipeline to the PET bottle line 8. In the PET bottle 11, a gas supply pipeline 16 and a gas exhaust pipeline 17 are inserted in the mouth portion so that gas is introduced from the mouth portion and gas is exhausted from the mouth portion repeatedly.

Further, PET bottle outside atmosphere adjusting means 31 connected by a pipeline sequentially to a standard gas cylinder 21 (e.g., Ar (99%) - $H_2$ (1%)), a pressure reducing valve 22, a mass flow controller 23 and PET bottle sealing means 24 such as a bag made of polypropylene are prepared in order to adjust the atmosphere outside the PET bottle (PET bottle) 11.

In the PET bottle sealing means 24 such as a bag made of polypropylene, when the PET bottle is covered and the PET bottle outside atmosphere adjusting means 31 is operated to make the atmosphere outside the PET bottle become Ar (99%) - $H_2$ (1%), for example, hydrogen which is the measurement object gas permeates from the outer surface of the PET bottle to the inner surface by diffuse permeation. Further, the hydrogen that has permeated to the inside of the plastic container is carried to the outside of the container by argon flowing through the PET bottle line 8 as a carrier gas, and the hydrogen concentration inside the argon is detected using the APIMS 15.

The means which heats the plastic molded body in the heat drying process can be illustrated by an apparatus (not shown in the drawings) in which the molded body is inserted in a heating chamber and blown with hot gas, an apparatus (not shown in the drawings) in which the plastic molded body is wound with a heater and wrapped by a heat conducting body such as aluminum foil or the like to heat the entire molded body or the like. Any apparatus may be used so long as it is possible to carry out overall heating at a level which does not cause heat deterioration or deformation of the plastic molded body.

With the apparatus shown in Fig. 3 forming a base, a description will be given for the operating procedure of the gas barrier property measuring method according to the present embodiment.

(1) Background Measurement

In the mass flow controller 6, argon gas is flowed (flowed to the bypass line 5) at 1 liter/minute and background data is measured. At this time, the valve A 13 is closed.

(2) Baking of the PET Bottle

The baking of the PET bottle 11 is carried out during the period of (1). For example, the entire container is heated by being wrapped by a heat conducting body such as aluminum foil or the like. In the mass flow controller 9, argon gas is flowed at 1 liter/minute in the PET bottle 11, and baking is carried out for 30 minutes at 80°C in the case of a heat resistant PET bottle. At this time, the valve B 12 is opened and gas is exhausted. Further, when flowing gas to the PET bottle 11, either the valve A 13 or the valve B 12 must be open. Further, as was described in the operation column, heat drying is carried out at or below a temperature level where shape deterioration and thin film deterioration such as peeling, cracking and like of the gas barrier property thin film do not occur, and preferably drying is carried out while blowing hot gas such as dry nitrogen gas, dry argon gas or the like in the container.

(3) Analysis of PET Bottle Line (room atmosphere)

In order to complete the heat drying process, the heater and the aluminum foil attached to the PET bottle 11 are removed. The valve B 12 is closed, the valve A 13 is opened, and the gas of the mass flow controller 6 (bypass line 5) is stopped to begin analysis of the PET bottle line 8. At this time, the PET bottle is surrounded by air (room atmosphere), and in this state, analysis is carried out for a prescribed period of time such as 1 hour, for example.

(4) Analysis of PET Bottle Line (Standard Gas: Ar (99%) - $H_2$ (1%) Atmosphere)

The PET bottle 11 covered with a polypropylene bag 24, Ar (99%) - $H_2$ (1%) is flowed at 500 cc/minute in the mass flow controller 23, and analysis is carried out for a prescribed period of time such as 1 hour, for example.
The description given above was for the purpose of describing the measuring method, and the combination shown in Table 1 may be used as a standard gas.

SPECIFIC EXAMPLES

Specific Example 1

In the specific examples, a heat resistant round type PET bottle having a height of 157 mm, a trunk diameter of 68 mm, a mouth diameter of 28 mm, a thickness of 0.35 mm, a volume of 350 ml and a surface area of 320 cm$^2$ formed a container for measurement. An apparatus which includes the gas system shown in Fig. 3 was used as a measuring apparatus. An APIMS (UG-240PN manufactured by Hitachi Tokyo Electronics Company) was used as a measurement object gas detector. Measurements were carried out in accordance with the operating procedures (1), (2) and (3) of the gas barrier property measuring method according to the present embodiment. The time when the atmosphere outside the container formed the room atmosphere (air atmosphere) formed Specific Example 1.

Specific Example 2

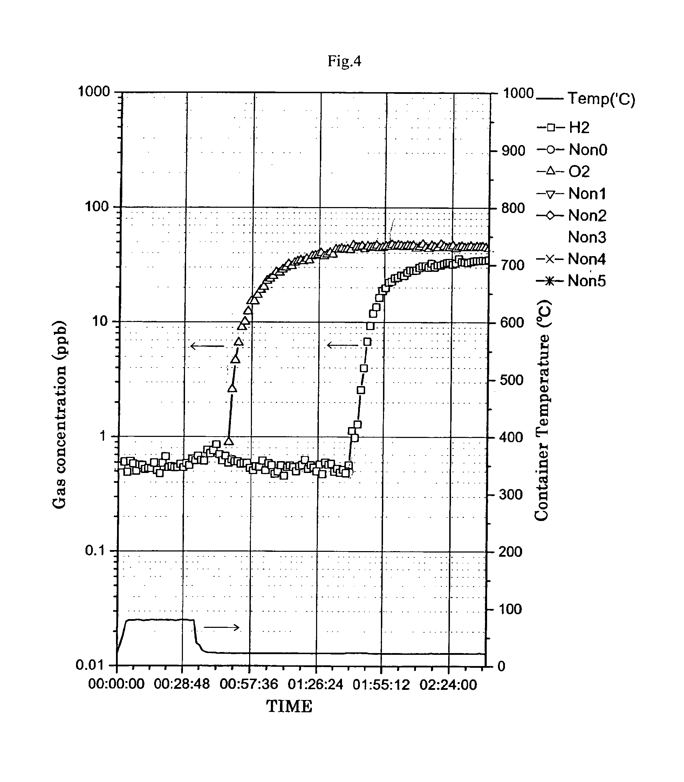

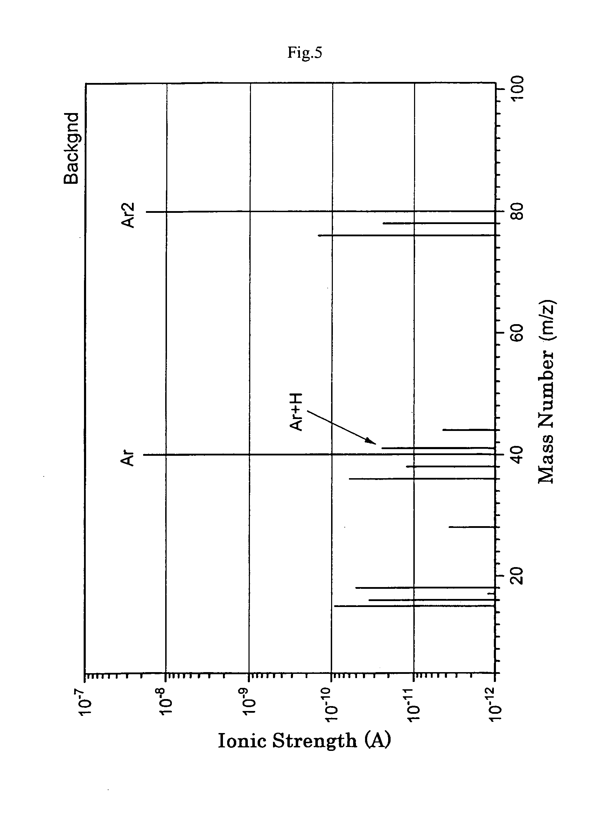

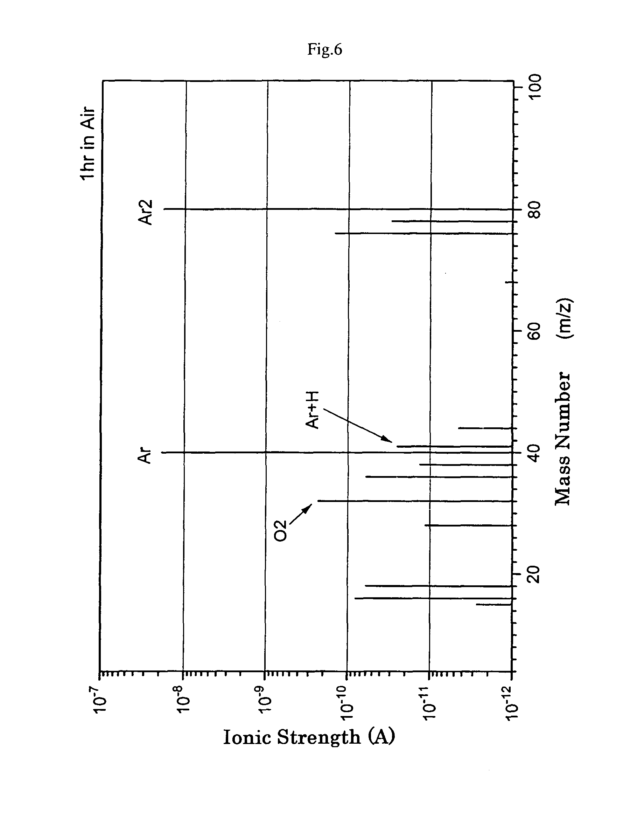

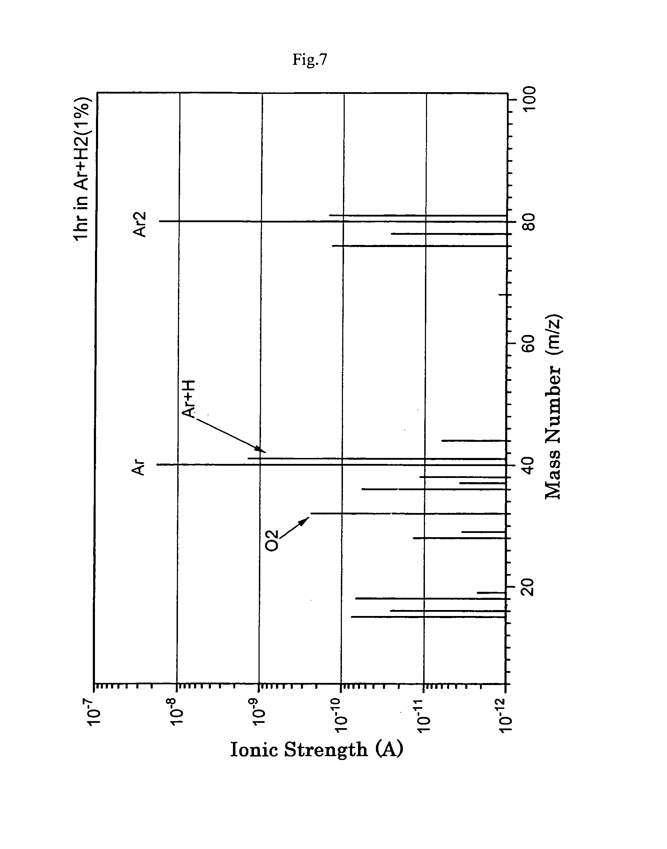

Continuous with Specific Example 1, the operating procedure (4) of the gas barrier property measuring method of the present embodiment was carried out, and the time when the atmosphere outside the container formed the standard gas: Ar (99%) - $H_2$ (1%) atmosphere formed Specific Example 2.
In Specific Examples 1 and 2, the changes in oxygen concentration and hydrogen concentration depending on the measurement elapsed time are shown in Fig. 4. Further, the relationship between the ionic strength and the mass number at the time of the background measurement of the operating procedure (1) of the gas barrier property measuring method is shown in Fig. 5. The relationship between the ionic strength and the mass number at the time of the oxygen concentration measurement of Specific Example 1 is shown in Fig. 6. The relationship between the ionic strength and the mass number at the time of the hydrogen concentration measurement of Specific Example 2 is shown in Fig. 7.

Specific Examples 3 and 4

A measurement object container was formed by a heat resistant round type PET bottle the same as that in Specific Example 1 in which a DLC film having a thickness of 9 nm was formed (at a film formation time of 0.9 seconds) uniformly on the inner surface. At this time, the output of the high frequency power supplied for film formation in a high frequency plasma CVD apparatus which was the same as that in Japanese Laid-Open Patent Application No. HEI 8-53116 was made 500 W. Measurements were carried out in the same way as in Specific Examples 1 and 2, wherein the time when the atmosphere outside the container formed the room atmosphere (air atmosphere) formed Specific Example 3 and the time when the atmosphere outside the container formed the standard gas: Ar (99%) - $H_2$ (1 %) atmosphere formed Specific Example 4.

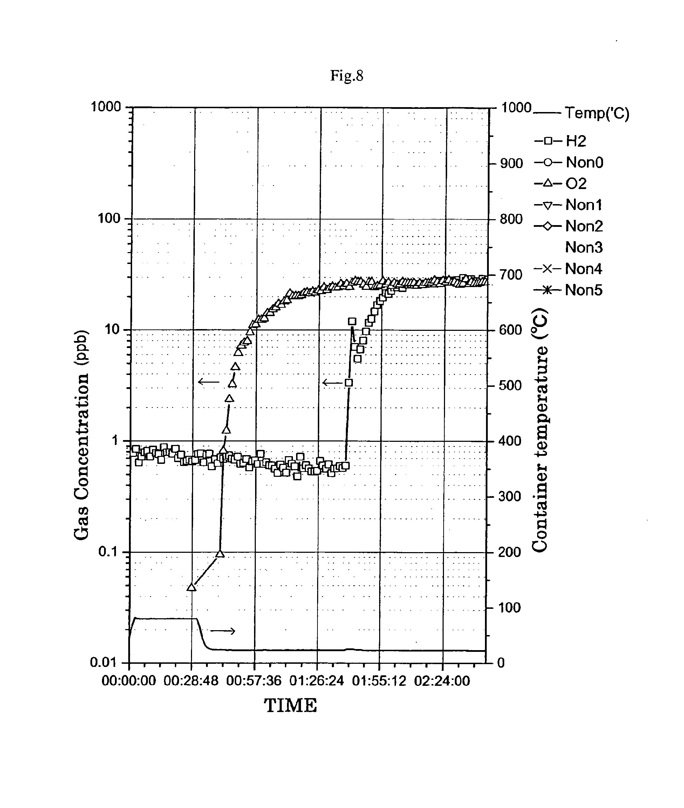

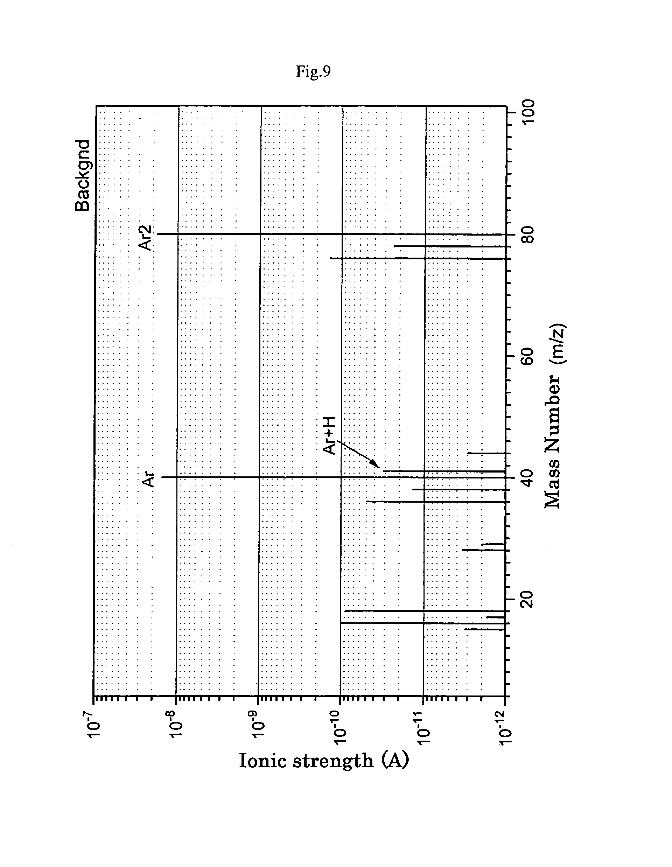

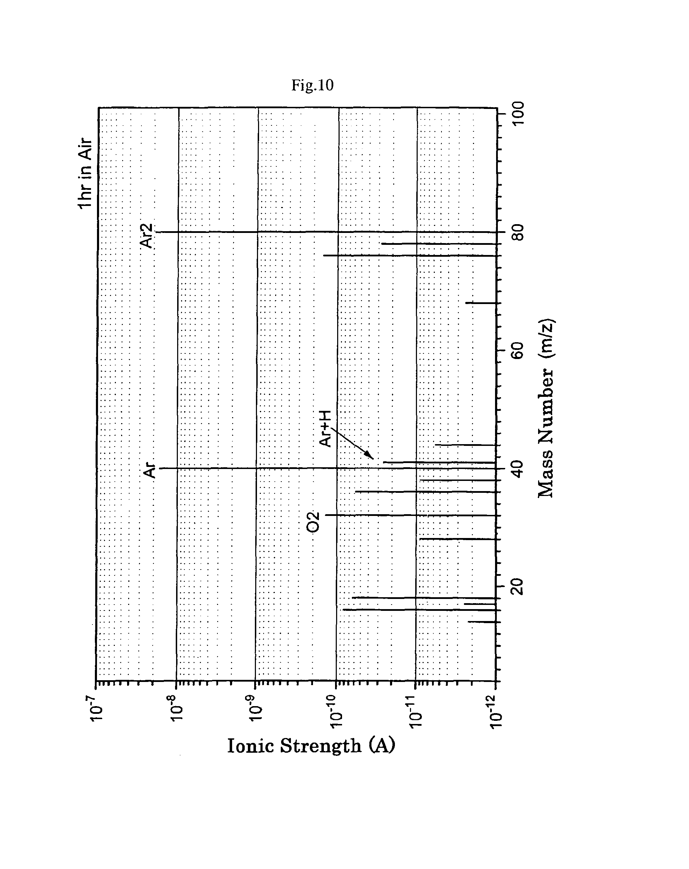

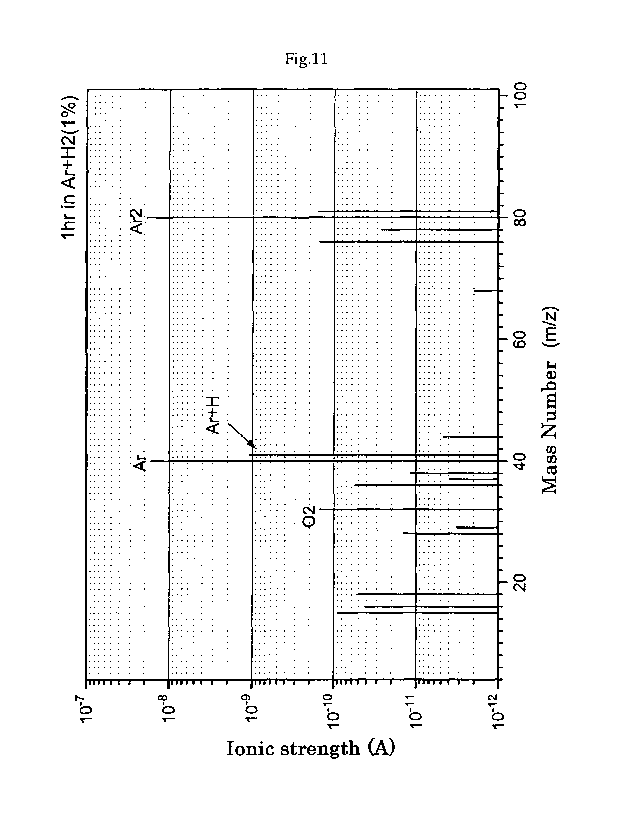

In Specific Examples 3 and 4, the changes in oxygen concentration and hydrogen concentration depending on the measurement elapsed time are shown in Fig. 8. Further, the relationship between the ionic strength and the mass number at the time of the background measurement of the operating procedure (1) of the gas barrier property measuring method is shown in Fig. 9. The relationship between the ionic strength and the mass number at the time of the oxygen concentration measurement of Specific Example 3 is shown in Fig. 10. The relationship between the ionic strength and the mass number at the time of the hydrogen concentration measurement of Specific Example 4 is shown in Fig. 11.

Specific Examples 5 and 6

A measurement object container was formed by a heat resistant round type PET bottle the same as that in Specific Example 1 in which a DLC film having a thickness of 14 nm was formed (at a film formation time of 1.4 seconds) uniformly on the inner surface. The film formation conditions were the same as in the case of Specific Examples 3 and 4. Measurements were carried out in the same way as in Specific Examples 1 and 2, wherein the time when the atmosphere outside the container formed the room atmosphere (air atmosphere) formed Specific Example 5 and the time when the atmosphere outside the container formed the standard gas: Ar (99%) - $H_2$ (1%) atmosphere formed Specific Example 6.

Specific Examples 7 and 8

A measurement object container was formed by a heat resistant round type PET bottle the same as that in Specific Example 1 in which a DLC film having a thickness of 19 nm was formed (at a film formation time of 1.9 seconds) uniformly on the inner surface. The film formation conditions were the same as in the case of Specific Examples 3 and 4. Measurements were carried out in the same way as in Specific Examples 1 and 2, wherein the time when the atmosphere outside the container formed the room atmosphere (air atmosphere) formed Specific Example 7 and the time when the atmosphere outside the container formed the standard gas: Ar (99%) - $H_2$ (1%) atmosphere formed Specific Example 8.

Specific Examples 9 and 10

A measurement object container was formed by a heat resistant round type PET bottle the same as that in Specific Example 1 in which a DLC film having a thickness of 24 nm was formed (at a film formation time of 2.4 seconds) uniformly on the inner surface. The film formation conditions were the same as in the case of Specific Examples 3 and 4. Measurements were carried out in the same way as in Specific Examples 1 and 2, wherein the time when the atmosphere outside the container formed the atmosphere (air atmosphere) inside the chamber formed Specific Example 9 and the time when the atmosphere outside the container formed the standard gas: Ar (99%) - $H_2$ (1%) atmosphere formed Specific Example 10.

The measured results of the amount of permeation of oxygen and hydrogen in Specific Examples 1 ~ 10 are shown in Table 3.

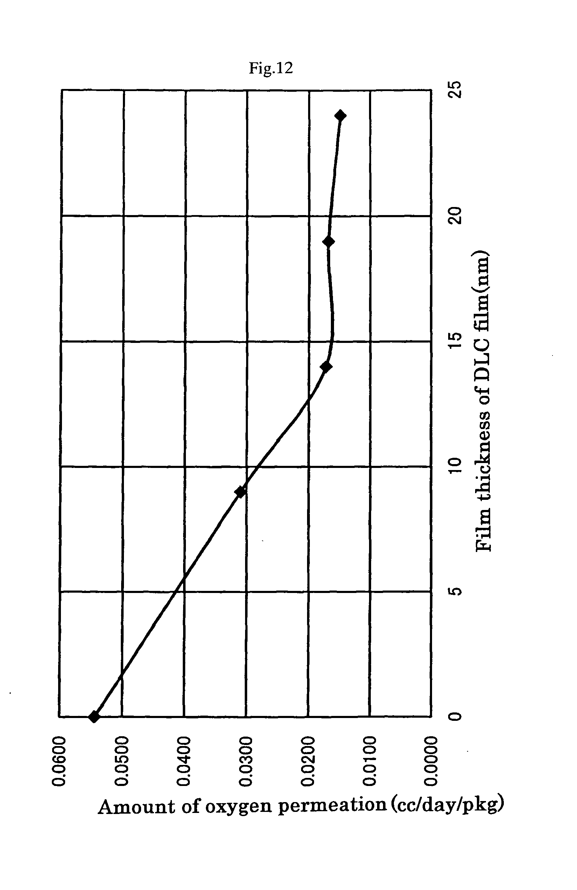

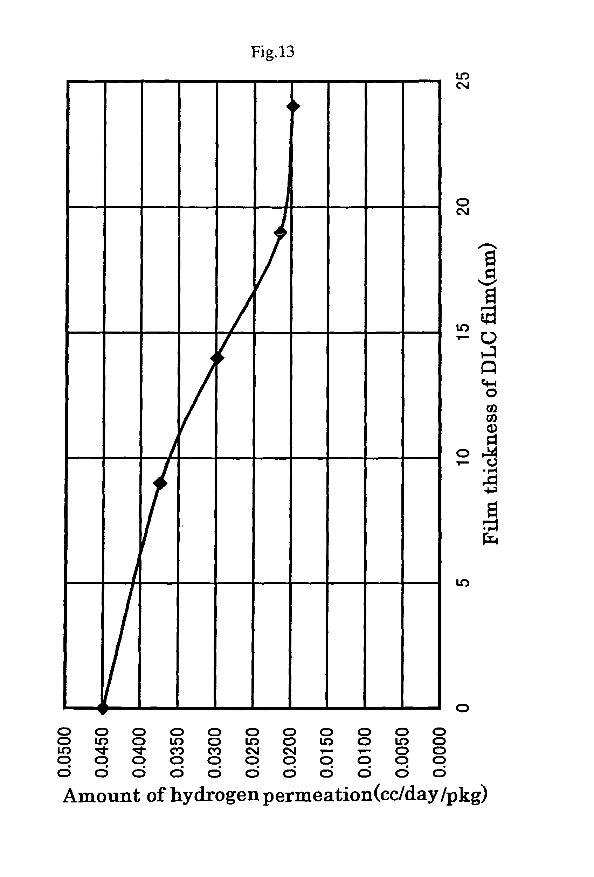

After the amount of permeation stabilized, the amount (ng) of permeation in 13 minutes is determined. This determined value is calculated based on the amount (cc/day/pkg) of permeation per one of each container. Further, the relationship between the film thickness of the DLC film and the amount of oxygen permeation is shown in Fig. 12, and the relationship between the film thickness of the DLC film and the amount of hydrogen permeation is shown in Fig. 13. Further, pkg is an abbreviation of Package.

TABLE 3

| Sample | Amount of Oxygen Permeation | | Amount of Hydrogen Permeation | |
|---|---|---|---|---|
| | Amount of Permeation in 13 minutes (ng) | Amount of Permeation per each 1 container (cc/day/pkg) | Amount of Permeation in 13 minutes (ng) | Amount of Permeation per each 1 container (cc/day/pkg) |
| Specific Example 1,2 | 701 | 0.0544 | 36.17 | 0.0449 |
| Specific Example 3,4 | 400.1 | 0.0310 | 30.16 | 0.0374 |
| Specific Example 5,6 | 222.53 | 0.0173 | 24.12 | 0.0299 |
| Specific Example 7,8 | 218.41 | 0.0169 | 17.29 | 0.0215 |
| Specific Example 9,10 | 191.87 | 0.0149 | 15.92 | 0.0198 |

As is understood from the results of Table 3, Fig. 12 and Fig. 13, it is clear that there is a relationship in which the amount of gas permeation becomes lower as the film thickness is made larger, and it was possible to evaluate the oxygen barrier property and the hydrogen barrier property. At this time, referring to Fig. 4 as an example, it took approximately 1.5 hours from the heat drying process to the completion of the oxygen barrier property measurement, and the total measurement time continued up to the completion of the hydrogen barrier property measurement was 2.5 hours, whereby it was possible to carry out measurements in a very short period of time. In Specific Examples 1 - 10, as is clear with reference to Fig. 4 or Fig. 8, for example, it is possible to measure the amount of oxygen gas permeation or the amount of hydrogen gas permeation in about 1.5 hours, and in the case where measurements of the amount of oxygen gas permeation and the amount of hydrogen gas permeation are continued, because the heat drying process can be omitted one time, evaluation can be carried out in 2.5 hours.

In Specific Examples 1 - 10, a method of measuring oxygen gas and hydrogen gas was shown, but by adjusting the atmosphere outside the container using a standard gas shown in Table 1, it is possible to carry out measurements in a short period of time in the same way as in the specific examples for the amount of gas permeation of carbon dioxide gas and oxygen gas (mass number 18).

Specific Example 11 and Comparative Example 1

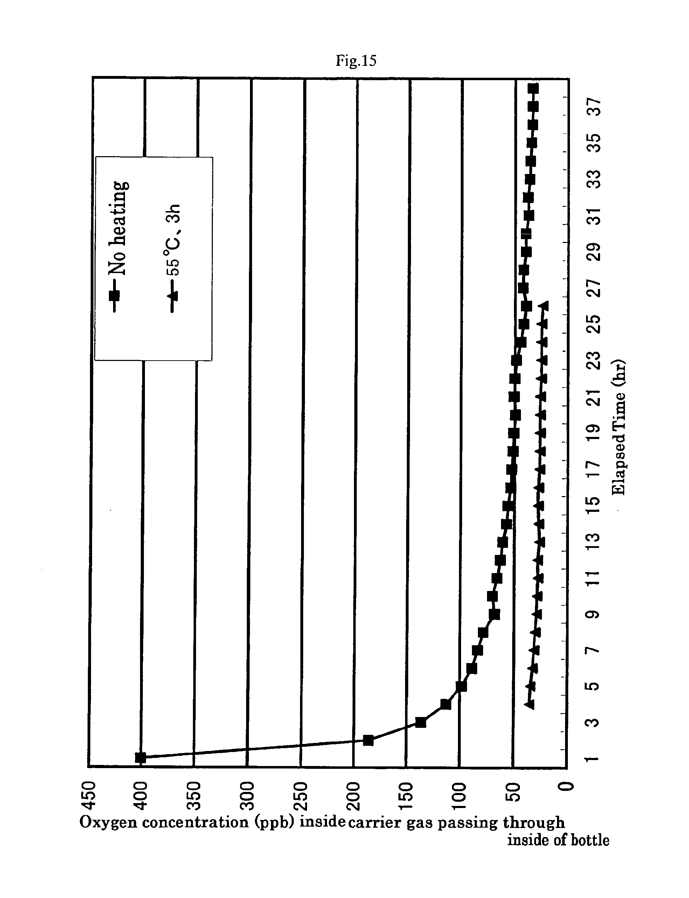

A comparison of the times required until the oxygen concentration stabilized was carried out by examining the change in oxygen concentration inside the carrier gas flowing through the inside of a PET container in the case where drying by heat drying was carried out and the case where it is not carried out. PET containers are containers which have a high hygroscopicity. Evaluation was carried out using the same containers as in Specific Example 1. The case where a plastic container underwent heat drying at 55°C for 3 hours formed Specific Example 11, and the case where heat drying was not carried out formed Comparative Example 1. Further, in Specific Example 11, argon gas was flowed through the inside of the container at a flow rate of 1000 ml/minute during heat drying. The outside of the container formed an air atmosphere. The results are shown in Fig. 15. Fig. 15 is a graph showing the change in oxygen concentration inside the carrier gas flowing through the inside of the PET container, and at the case where there is a heat drying process and the case where there is none. As is clear with reference to Fig. 15, in Specific Example 11, from the time measurements are begun (the time where 4 hours have elapsed in the drawing), the oxygen concentration is fixed at roughly 40 ppb, and after the heat drying process, it is possible to immediately carry out evaluation of the oxygen permeability. On the other hand, in Comparative Example 1, the oxygen concentration is lowered gradually as time elapses, and the oxygen concentration finally reaches roughly 40 ppb at the time when 38 hours have elapsed in the drawing. Accordingly, the time required until the beginning of measurements is clearly short in Specific Example 11 which undergoes a heat drying process. Accordingly, the effect of having a heat drying process became clear.

Specific Example 12 and Comparative Example 2

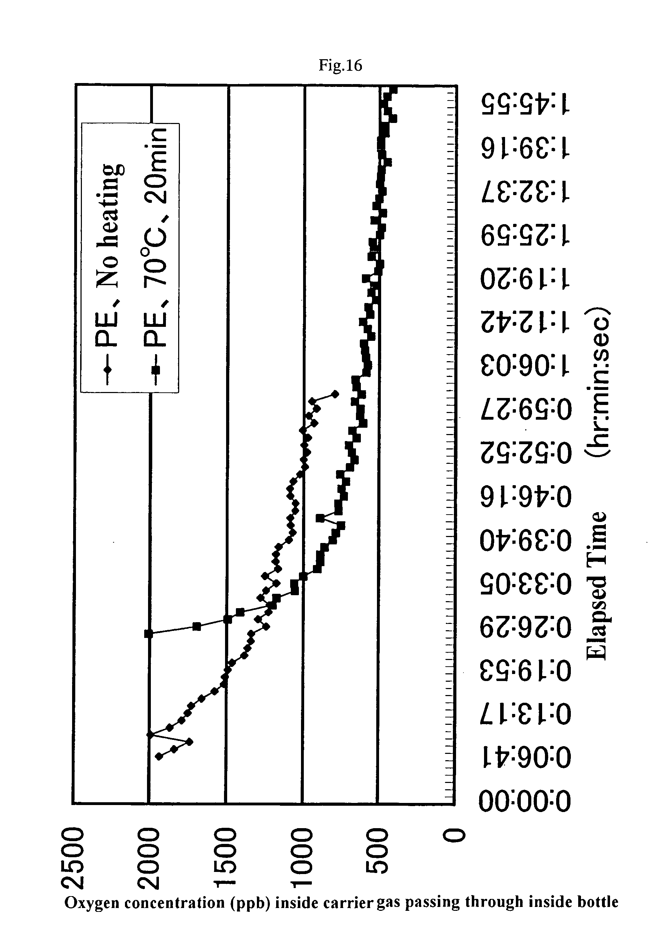

A comparison of the times required until the oxygen concentration stabilized was carried out by examining the change in oxygen concentration inside the carrier gas flowing through the inside of a polyethylene container in the case where drying by heat drying was carried out and the case where it is not carried out. Polyethylene containers are containers which have a low hygroscopicity. Evaluation was carried out with containers having the same size as the containers in Specific Example 1. The case where a plastic container underwent heat drying at 70°C for 20 minutes formed Specific Example 12, and the case where heat drying was not carried out formed Comparative Example 2. Further, in Specific Example 12, argon gas was flowed through the inside of the container at a flow rate of 1000 ml/minute during heat drying. The outside of the container formed an air atmosphere. The results are shown in Fig. 16. Fig. 16 is a graph showing the change in oxygen concentration inside the carrier gas flowing through the inside of the polyethylene container, and at the case where there is a heat drying process and the case where there is none. With reference to Fig. 16, in Specific Example 12, the oxygen concentration is lowered gradually as time elapses from the time measurements are begun (the time where 26 minutes have elapsed in the drawing). On the other hand, in Comparative Example 2, the oxygen concentration is lowered gradually as time elapses from the time measurements are begun (the time where 6 minutes have elapsed in the drawing). However, in Specific Example 12, the oxygen concentration is lower than even Comparative Example 10, and the oxygen permeability stabilizes quickly. Accordingly, even in a polyethylene container which is a container having a low hygroscopicity, it is possible to shorten the time required until measurements are begun by providing a heat drying process. Accordingly, the effect of having a heat drying process became clear.

Comparative Example 3

A heat resistant round type PET bottle (non-coated bottle) which was the same as that in Specific Example 1 formed the measurement container. Instead of carrying out the heat drying process of the operating procedure (2) of the gas barrier property according to the present embodiment, oxygen concentration measurements were carried out after argon was flowed at a flow rate of 1 liter/minute for 10 hours without heating, and this formed Comparative Example 3.
In Comparative Example 3, the values finally stabilized after argon was flowed at 1000 cc/minute for 10 hours. Compared with Specific Example 1, a long time was required for drying to remove water molecules, and quick measurements were impossible.

Comparative Examples 4, 5, 6, 7 and 8

Using an OX-TRAN2/21 manufactured by Mocon Company, measurements of the amount of oxygen permeation (cc/day/pkg) were carried out for the non-coated plastic container measured in Specific Examples 1 and 2, the coated plastic container that underwent film formation for 0.9 seconds measured in Specific Examples 3 and 4, the coated plastic container that underwent film formation for 1.4 seconds measured in Specific Examples 5 and 6, the coated plastic container that underwent film formation for 1.9 seconds measured in Specific Examples 7 and 8, and the coated plastic containers that underwent film formation for 2.4 seconds measured in Specific Examples 9 and 10 with the inside of the containers at 23°C - 55%RH, the outside of the containers at 23°C - 100%RH, and the oxygen partial pressure at 21%. These sequentially formed Comparative Example 4, Comparative Example 5, Comparative Example 6, Comparative Example 7 and Comparative Example 8 as shown in Table 4. As for the measurement times, measurements were carried out after 1 day, after 2 days, after 3 days, after 4 days, after 5 days and after 6 days, respectively. These results are shown in Table 5.

TABLE 4

| Sample | Film Formation Time (seconds) | DLC Film Thickness (nm) |
|---|---|---|
| Comparative Example 4 | 0 | 0 |
| Comparative Example 5 | 0.9 | 9 |
| Comparative Example 6 | 1.4 | 14 |
| Comparative Example 7 | 1.9 | 19 |
| Comparative Example 8 | 2.4 | 24 |

TABLE 5

| Sample | Day Number | | | | | |
|---|---|---|---|---|---|---|
| | 1 | 2 | 3 | 4 | 5 | 6 |
| Comparative Example 4 | 0.0324 | 0.0265 | 0.0251 | 0.0247 | 0.0239 | 0.0244 |
| Comparative Example 5 | 0.0142 | 0.0123 | 0.0113 | 0.0113 | 0.0107 | 0.0107 |
| Comparative Example 6 | 0.0077 | 0.0068 | 0.0060 | 0.0057 | 0.0054 | 0.0055 |
| Comparative Example 7 | 0.0062 | 0.0056 | 0.0054 | 0.0053 | 0.0051 | 0.0052 |
| Comparative Example 8 | 0.0050 | 0.0043 | 0.0042 | 0.0040 | 0.0040 | 0.0040 |
| Units : Amount of permeation per each 1 container (cc/day/pkg) | | | | | | |

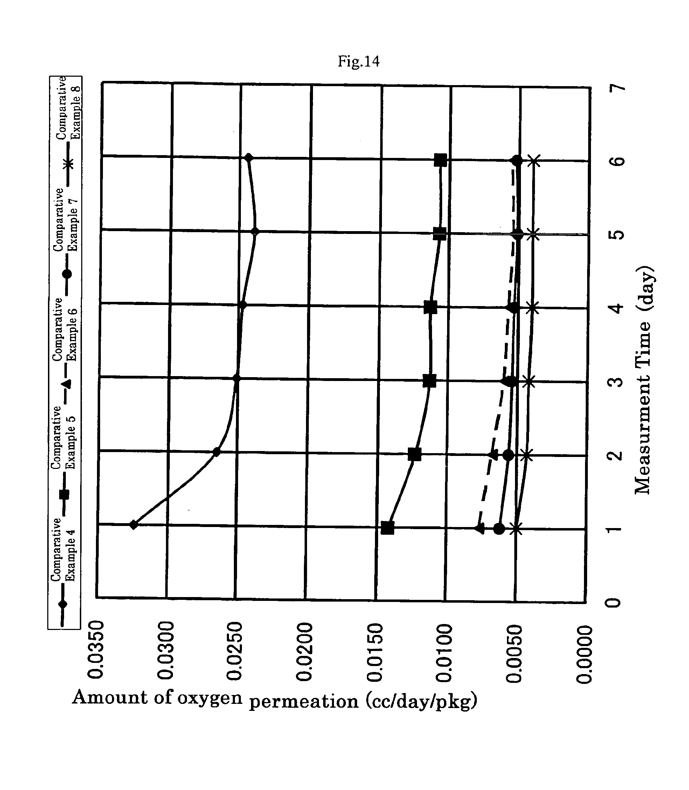

The relationship between the measurement elapsed time and the amount of oxygen permeation in Comparative Examples 4 - 8 is shown in Fig. 14.

In Comparative Examples 4~8, there is no provision of a heat drying process, and it is not possible to flow a large amount of argon gas as a carrier gas. At this time, a long period of time is required until the measurement data stabilizes. During this long period of time, it is assumed that water molecules are removed, and the inside of the container undergoes gas substitution. As is clear with reference to Fig. 14, approximately 5~6 days was required until the amount of oxygen permeation stabilized. It was understood that the evaluation time was very long compared with the fact that it was possible to complete measurements of the amount of oxygen permeation in 1.5 hours after the beginning of measurements in Specific Examples 1, 3, 5, 7 and 9.

**Claims**

1.    A method of measuring the gas barrier property of a plastic molded body such as a plastic container, a plastic sheet or a plastic film or the like which uses a gas analyzer to measure the amount of permeation of a measurement object gas permeating through the plastic molded body, comprising:

a heat drying process which heats and dries said plastic molded body in a temperature range which does not cause deformation or heat deterioration.

2.    The method of measuring the gas barrier property of a plastic molded body described in Claim 1, further comprising an atmosphere adjusting process which forms an atmosphere including a measurement object gas at one side of the wall surfaces of said plastic molded body, and forms a carrier gas atmosphere which carries said measurement object gas after permeation to said gas analyzer at the other side forming a front and back relationship of said wall surfaces.

3.    The method of measuring the gas barrier property of a plastic molded body described in Claim 1 or 2, wherein said carrier gas atmosphere is an argon atmosphere or a nitrogen gas atmosphere.

4.    The method of measuring the gas barrier property of a plastic molded body described in Claim 1, 2 or 3, wherein the heat drying process is carried out at the same time in the atmosphere adjusting process.

5.    The method of measuring the gas barrier property of a plastic molded body described in Claim 1, 2, 3 or 4, further comprising a measurement object gas measuring process which measures the amount of permeation of said measurement object gas permeating through said plastic molded body from one side of said wall surfaces toward the other side with said gas analyzer at the time when said amount of permeation of said measurement object gas reaches a roughly steady state.

6.    The method of measuring the gas barrier property of a plastic molded body described in Claim 1, 2, 3, 4 or 5, wherein said heat drying process includes a process which removes water absorbed by said plastic molded body.

7.    The method of measuring the gas barrier property of a plastic molded body described in Claim 1, 2, 3, 4, 5 or 6, wherein said heat drying process includes a process which substitutes adsorption gas adsorbed on one side of the wall surfaces of said plastic molded body with gas of the atmosphere including said measurement object gas, and substitutes adsorption gas adsorbed on the other side forming a front and back relationship of the wall surfaces of said plastic molded body with gas of said carrier gas atmosphere.

8. The method of measuring the gas barrier property of a plastic molded body described in Claim 1, 2, 3, 4, 5, 6 or 7, wherein said gas analyzer is a mass spectrometer.

9. The method of measuring the gas barrier property of a plastic molded body described in Claim 1, 2, 3, 4, 5, 6, 7 or 8, wherein said mass spectrometer is an atmospheric pressure ionization mass spectrometer (APIMS).

10. The method of measuring the gas barrier property of a plastic molded body described in Claim 1, 2, 3, 4, 5, 6, 7, 8 or 9, wherein said plastic molded body is a plastic container or a gas barrier property plastic container in which a gas barrier property thin film is coated on one of the inner surface or the outer surface or both surfaces thereof, and an atmosphere including a measurement object gas is formed outside the container and said carrier gas atmosphere is formed inside the container in said atmosphere adjusting process.

11. The method of measuring the gas barrier property of a plastic molded body described in Claim 1, 2, 3, 4, 5, 6, 7, 8, 9 or 10, wherein said measurement object gas is oxygen gas, and the atmosphere including said measurement object gas is an air atmosphere.

12. The method of measuring the gas barrier property of a plastic molded body described in Claim 1, 2, 3, 4, 5, 6, 7, 8, 9 or 10, wherein said measurement object gas is one of oxygen gas, carbon dioxide gas or hydrogen gas or a combination thereof, and the atmosphere including said measurement object gas is a measurement object gas - argon atmosphere or a measurement object gas - nitrogen gas atmosphere in which adjustments are carried out so that said measurement object gas forms a prescribed partial pressure.

13. The method of measuring the gas barrier property of a plastic molded body described in Claim 1, 2, 3, 4, 5, 6, 7, 8, 9 or 10, wherein said measurement object gas is oxygen gas of mass number 18, and the atmosphere including said measurement object gas is an oxygen gas of mass number 18 - argon atmosphere or an oxygen gas of mass number 18 - nitrogen gas atmosphere in which adjustments are carried out so the oxygen gas of mass number 18 forms a prescribed partial pressure.

14. The method of measuring the gas barrier property of a plastic molded body described in Claim 1, 2, 3, 4, 5, 6, 7, 8, 9 or 10, wherein said measurement object gas is a mixed gas of oxygen gas of mass number 18 and at least one gas from oxygen gas of mass number 16, carbon dioxide gas or hydrogen gas, and the atmosphere including said measurement object gas is a measurement object gas - argon atmosphere or a measurement object gas - nitrogen gas atmosphere in which adjustments are carried out so that said measurement object gas forms a prescribed partial pressure.

15. The method of measuring the gas barrier property of a plastic molded body described in Claim 1, 2, 3, 4, 5, 6, 7, 8, 9, 10, 11, 12, 13 or 14, wherein said plastic container is a gas barrier property plastic container which is mass produced by arranging a plurality of coating chambers for coating the inner surface or the outer surface of the plastic container or both surfaces thereof with a gas barrier property thin film and operating said coating chambers simultaneously or sequentially, said heat drying process, said atmosphere adjusting process and said measurement object gas measuring process are carried out at a level where a prescribed number of film formations is carried out in each of said coating chambers, and then a chamber operation judgment process which judges poor operation of said coating chambers by comparing the gas barrier properties of said gas barrier property plastic containers in each of said coating chambers is carried out.

16. The method of measuring the gas barrier property of a plastic molded body described in Claim 1, 2, 3, 4, 5, 6, 7, 8, 9, 10, 11, 12, 13, 14 or 15, wherein the argon flow rate per 1 minute or the nitrogen flow rate per 1 minute supplied to said carrier gas atmosphere side is greater than or equal to twice the volume of said plastic container in said atmosphere adjusting process and said measurement object gas measuring process.

Fig.1

Source Gas Supply Means

Rotational
Support
Body

Film
Formation
Chamber

Containers
Prior to Film
Formation

Containers after Film
Formation

High Frequency Means

Fig.2

Gas adjustment process
area before film
formation
(Stabilization area)

22.5°

0°

135°

CVD film formation area

Gas adjustment
process area
before film
formation

101.25°

Container
loading
area

Container
unloading
area

Gas adjustment
process area after
film formation

11.25°

Containers prior to film
formation

Containers after film formation

Fig.3

Fig.4

Fig.5

Fig.6

EP 1 602 912 A1

Fig.7

Fig.8

Fig.9

Fig.10

Fig.11

Fig.12

Fig.13

Fig.14

Fig.15

Fig.16

<table>
<tr><td colspan="2" align="center">**INTERNATIONAL SEARCH REPORT**</td><td>International application No.<br>PCT/JP2004/002223</td></tr>
</table>

| | |
|---|---|
| A. CLASSIFICATION OF SUBJECT MATTER<br>    Int.Cl⁷ G01M13/00, G01N15/08 | |

According to International Patent Classification (IPC) or to both national classification and IPC

**B. FIELDS SEARCHED**

Minimum documentation searched (classification system followed by classification symbols)
    Int.Cl⁷ G01M13/00, G01N15/08, G01M3/00, B29C, B65D

Documentation searched other than minimum documentation to the extent that such documents are included in the fields searched
    Jitsuyo Shinan Koho         1922–1996   Toroku Jitsuyo Shinan Koho    1994–2004
    Kokai Jitsuyo Shinan Koho   1971–2004   Jitsuyo Shinan Toroku Koho    1996–2004

Electronic data base consulted during the international search (name of data base and, where practicable, search terms used)
        JICST(JOIS), WPI

**C. DOCUMENTS CONSIDERED TO BE RELEVANT**

| Category* | Citation of document, with indication, where appropriate, of the relevant passages | Relevant to claim No. |
|---|---|---|
| A | WO 01/69201 A1  (TETRA LAVAL HOLDINGS & FINANCE S.A.),<br>20 September, 2001 (20.09.01),<br>Page 4, lines 27 to 34<br>& EP 1269150 A        & DE 10012446 A<br>& AU 5219501 A       & US 2003-46982 A | 1-16 |
| A | WO 96/05111 A  (Kirin Brewery Co., Ltd.),<br>22 February, 1996 (22.02.96),<br>Full text; all drawings<br>& EP 773166 A1       & DE 69528982 T<br>& US 6589619 B       & CA 2196888 A<br>& AT 228462 T        & DK 773166 T<br>& JP 8-53116 A | 1-16 |

☒ Further documents are listed in the continuation of Box C.      ☐ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    07 May, 2004 (07.05.04) | Date of mailing of the international search report<br>    25 May, 2004 (25.05.04) |
|---|---|
| Name and mailing address of the ISA/<br>    Japanese Patent Office | Authorized officer |
| Facsimile No. | Telephone No. |

Form PCT/ISA/210 (second sheet) (January 2004)

# EP 1 602 912 A1

| INTERNATIONAL SEARCH REPORT | International application No. |
|---|---|
| | PCT/JP2004/002223 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-214069 A (Sony Corp.),<br>04 August, 2000 (04.08.00),<br>Full text; all drawings<br>(Family: none) | 1-16 |
| A | JP 6-241978 A (Mitsui Toatsu Chemicals, Inc.),<br>02 September, 1994 (02.09.94),<br>Full text; all drawings<br>(Family: none) | 1-16 |
| A | WO 96/26426 A1 (Osaka Oxygen Industies, Ltd.),<br>29 August, 1996 (29.08.96),<br>Claims<br>& US 5827949 A | 9 |

Form PCT/ISA/210 (continuation of second sheet) (January 2004)